# FASCICULE DE BREVET EUROPEEN

(11) **EP 2 996 657 B1**
(45) Date de publication et mention de la délivrance du brevet: **22.03.2017**
(21) Numéro de dépôt: 14723787.9
(22) Date de dépôt: 13.05.2014
(51) Int. Cl.: A61G 9/00, A61F 5/44, B65D 33/00

(54) **POCHE SOUPLE A MANCHON D'OUVERTURE A BEC**
FLEXIBLER BEUTEL MIT ÖFFNUNGSHÜLSE MIT AUSGUSS
FLEXIBLE BAG WITH OPENING SLEEVE WITH SPOUT

(30) Priorité: 13.05.2013 FR 1354249
(43) Date de publication de la demande: 23.03.2016
(73) Titulaire: M3AT SA, 1652 Botterens (CH)
(72) Inventeur: CAILLETEAU, Benoît, 13007 Marseille (FR)
(74) Mandataire: Intès, Didier Gérard André
(86) Numéro de dépôt international: PCT/EP2014/059724
(87) Numéro de publication internationale: WO 2014/184174

(56) Documents cités:
- EP-A1- 0 847 742
- JP-A- 2003 125 975
- US-A1- 2010 318 044

## Description

La présente invention concerne une poche comprenant une enveloppe souple ayant une ouverture dont le bord est fixé aux deux parois opposées d'un manchon de renfort en saillie à l'extérieur de l'enveloppe, la poche étant susceptible d'adopter une configuration aplatie dans laquelle le manchon est plié selon une ligne de pliage principale reliant ses parois de sorte que lesdites parois sont situées l'une contre l'autre, et une configuration d'utilisation dans laquelle ces parois forment un canal qui maintient l'ouverture ouverte.

Des poches de ce type sont connues, par exemple par le brevet européen n° 0 847 742. Elles sont utilisées pour recevoir des produits sous forme généralement liquide, notamment des déchets d'origine humaine ou animale tels que de l'urine. Ces poches sont stockées dans leur configuration aplatie. Pour leur utilisation, le manchon est manipulé de manière à ce que ses parois forment le canal qui maintient l'ouverture ouverte, mettant ainsi la poche dans sa configuration d'utilisation, dans laquelle les déchets peuvent être déversés à l'intérieur de cette poche. Une fois que les déchets ont été introduits, il suffit de relâcher le manchon pour que la poche reprenne sa configuration aplatie.

Ces poches sont couramment réalisées au moyen de feuilles minces de matière plastique ou autre matériaux, éventuellement transparentes, dotées de la souplesse voulue. Pour l'ouverture, la manipulation du manchon consiste à déformer ses parois de manière à les contraindre à adopter une forme convexe (vue de l'extérieur de la poche) dans laquelle elles forment entre elles le canal qui maintient l'ouverture ouverte.

La demande de brevet européen n° 0 847 742 s'intéresse au fait d'assurer que la manipulation du manchon le conformant en canal provoque l'écartement des parois de l'enveloppe, non seulement sur les bords de l'ouverture de l'enveloppe, mais également en aval de cette ouverture, pour éviter que le produit introduit dans la poche n'ait tendance à refluer. Pour cela, le manchon est doté de pattes internes d'écartement. Ceci donne une grande liberté de choix quant au matériau constituant l'enveloppe de la poche. En effet, grâce à l'invention objet de la demande de brevet européen précitée, on assure que les feuilles de l'enveloppe s'écartent même si elles sont très souples, voire si elles ont initialement une tendance à rester légèrement collées.

Un problème posé à l'élaboration de poche du type précité est celui d'assurer une conduction efficace des produits (en particulier des déchets tels que de l'urine) à l'intérieur de l'enveloppe. Lorsque la poche est utilisée comme poche à urine à usage masculin, cette conduction ne pose normalement pas de difficulté particulière, dès lors que la verge de l'utilisateur est introduite à travers l'ouverture (dans le canal formé par le manchon) sur une longueur suffisante. De même, pour toutes les applications au recueillement de déchets, dans lesquelles les déchets sont introduits dans la poche à partir d'un organe ou d'embout saillant pouvant être introduit dans la poche à travers l'ouverture sur une longueur suffisante, la conduction des produits à l'intérieur de la poche ne pose généralement pas de difficulté.

En revanche, des difficultés existent lorsque l'on souhaite utiliser les poches pour d'autres applications dans lesquelles l'organe à partir duquel les produits s'écoulent n'est pas du type pouvant être introduit dans le canal formé par le manchon, ou ne peut pas y être introduit sur une longueur suffisante. C'est par exemple le cas lorsque les poches sont utilisées comme poches à urine à usage féminin. En effet, dans ce cas, les produits, par exemple l'urine, sont introduits dans la poche à partir de l'extrémité du manchon qui fait saillie à l'extérieur de l'enveloppe.

La demanderesse a constaté que, lorsque le bord libre du manchon présente la forme décrite dans la demande de brevet européen n° 0 847 742, le guidage des produits à partir de ce bord libre peut être insuffisant. C'est en particulier le cas lorsque la poche est utilisée comme urinal à usage féminin.

La demande de brevet FR 2 735 358 concerne spécifiquement un urinal à usage féminin dont le bord libre est censé présenter une forme adaptée à l'anatomie féminine. Cependant, cet urinal est totalement rigide. Contrairement à la poche, objet de la présente invention, il ne peut donc pas être stocké dans un encombrement limité.

La demande de brevet FR 2 898 269 concerne une poche formant un urinal à usage féminin, ayant une collerette dite ergonomique, censée présenter une forme adaptée à l'anatomie féminine. Cependant, cette collerette est en elle-même rigide et ne peut donc pas être aplatie, ce qui pénalise également les capacités de stockage.

Le document US 2010/0318044 A1 présente une poche conforme au préambule de la revendication 1.

L'invention vise à remédier aux inconvénients précités en proposant une poche qui puisse adopter une configuration aplatie et dont le manchon présente une conformation favorisant le guidage des produits introduits dans la poche vers le fond de cette dernière, en limitant les risques de fuite, en particulier lorsque la poche est utilisée comme urinal à usage féminin.

Ce but est atteint grâce au fait que la ligne de pliage principale présente une portion d'extrémité opposée à l'ouverture qui est en avancée latérale vers l'extérieur, de sorte que, en configuration d'utilisation, le manchon présente un bec délimité par ladite portion d'extrémité.

Grâce à ces dispositions, lorsque le manchon est manipulé pour passer dans sa configuration d'utilisation, le bec se forme naturellement. Ce bec favorise le guidage des produits vers l'intérieur de la poche. En particulier, lorsque la poche est utilisée comme urinal féminin, le bec peut être disposé contre la partie postérieure de la vulve, limitant ainsi les risques de fuites d'urine entre l'anatomie féminine et la poche.

La poche selon l'invention trouve de nombreuses applications. En effet, du fait de la présence du bec, la conduction des produits le long du canal dans la poche est améliorée, ce qui est en particulier utile lorsque, de manière générale, les produits s'écoulent à partir d'organes ne pouvant pas être introduits dans la poche. Elle est également utile lorsque les produits s'écoulent à partir d'organes qui peuvent être introduits dans la poche, puisque l'invention permet d'éviter les fuites sans qu'il ne soit nécessaire d'apporter un soin particulier à la profondeur d'introduction des organes en question dans la poche.

La ligne de pliage principale présente une concavité tournée vers l'extérieur du manchon.

Lors du passage du manchon de sa configuration aplatie à sa configuration d'utilisation, cette concavité produit un effet de voûte qui contraint le bec à se former naturellement, en favorisant le « dépliage » selon la ligne de pliage.

Avantageusement, chacune des parois du manchon présente une ligne de pliage secondaire, qui relie la ligne de pliage principale au bord libre du manchon opposé à l'ouverture et qui, en configuration d'utilisation, forme une démarcation entre le bec et une partie restante du manchon.

Lors du passage du manchon de sa configuration aplatie à sa configuration d'utilisation, les parois du manchon ont tendance à se creuser selon les lignes de pliage secondaires, ce qui favorise encore la mise en forme du bec.

Avantageusement, les lignes de pliage secondaires sont symétriques par rapport à la ligne de pliage principale.

Le bec est alors lui-même formé symétriquement.

Avantageusement, les lignes de pliage secondaires sont courbes, de concavité tournée du côté de la ligne de pliage principale.

Ceci favorise la mise en forme du bec en saillie latérale vers l'extérieur.

Avantageusement, la hauteur de dépassement du manchon par rapport à l'ouverture varie sur la largeur du manchon, entre une hauteur minimale mesurée au voisinage de la ligne de pliage principale et une hauteur maximale mesurée au voisinage de l'extrémité latérale du manchon opposée à la ligne de pliage principale.

Cette variation de hauteur est particulièrement intéressante lorsque la poche est utilisée comme urinal à usage féminin, car elle favorise le placage du bord libre du manchon contre la vulve de la femme qui utilise la poche, la partie de plus faible hauteur, qui comporte le bec, étant disposée contre la partie postérieure de la vulve.

Avantageusement, la première paroi du manchon présente une première bande longitudinale d'extrémité en saillie latérale par rapport au bord de l'ouverture, tandis que la deuxième paroi présente un bord de glissement apte à glisser contre la face interne de ladite première bande longitudinale lors du passage de la poche de sa configuration aplatie à sa configuration d'utilisation.

Ainsi, lorsque l'on presse les bords longitudinaux du manchon de manière à les rapprocher l'un de l'autre, on déforme de manière certaine la première paroi du manchon en la rendant convexe, grâce au fait que la première bande longitudinale d'extrémité est en saillie latérale par rapport aux bords de l'ouverture. Cette première paroi étant rendue convexe, le bord de glissement de la deuxième paroi n'a pas d'autre choix que de glisser contre la face interne, qui est donc concave, de la première bande longitudinale. En conséquence, la deuxième paroi s'écarte de la première paroi, de sorte que ces deux parois forment entre elles le canal souhaité pour maintenir l'ouverture ouverte. De plus, le contact de glissement entre le bord de glissement et la deuxième paroi procure une certaine étanchéité évitant, lorsque les déchets sont introduits dans la poche, les fuites de ceux-ci par le bord du canal pourvu du bord de glissement et de la première bande longitudinale souhaitée.

En particulier, le manchon peut être fabriqué par moulage de matière plastique, par exemple par injection, sous la forme d'une seule pièce présentant les deux parois, reliées par la ligne de pliage principale. On peut également fabriquer le manchon à partir de deux flancs découpés à plat et formant respectivement chacune des parois, ces flancs étant ensuite reliés (par soudage ou analogue) selon la ligne de pliage principale.

Avec la présente invention, une grande liberté de forme est laissée en ce qui concerne le bord libre du manchon opposé à l'ouverture. Ce bord peut être rectiligne, par exemple lorsque la poche est utilisée comme urinal à usage masculin. Pour d'autres applications, par exemple lorsque la poche est utilisée comme urinal à usage féminin, une forme différente peut être donnée, ainsi qu'il a été indiqué.

Avantageusement, la poche comprend des moyens pour maintenir les parois du manchon dans leur conformation formant un canal.

Par exemple, le manchon comporte deux languettes de maintien, respectivement portées par la première et par la deuxième paroi du manchon, du côté opposé à l'ouverture de la poche et dans une zone éloignée de la ligne de pliage principale, les languettes de maintien se recouvrant au moins partiellement dans la position d'utilisation.

Ces languettes de maintien forment ainsi une zone de préhension, par laquelle le manchon conformé en canal peut être saisi et maintenu dans sa conformation en canal.

On peut également prévoir que deux parties du manchon, destinées à venir en recouvrement lorsque les parois du manchon sont dans leur conformation formant un canal, présentent respectivement un perçage et un ergot apte à être engagé dans ledit perçage.

Ces deux parties destinées à venir en recouvrement sont avantageusement situées dans des zones respectives de la première et de la deuxième paroi du manchon qui sont éloignées de la ligne de pliage principale.

L'invention sera bien comprise et ses avantages apparaîtront mieux à la lecture de la description détaillée qui suit, d'un mode de réalisation représenté à titre d'exemple non limitatif. La description se réfère aux dessins annexés sur lesquels :
- la figure 1 est une vue en plan d'une poche conforme à l'invention, dans sa configuration aplatie ;
- la figure 2 est une vue en perspective montrant le manchon de la poche de la figure 1 ;
- la figure 3 est une vue en plan montrant les deux parois du manchon de la figure 1, selon une variante ;
- la figure 4 est une vue en perspective montrant la forme du manchon en configuration d'utilisation de la poche de la figure 1 ;
- la figure 5 est une vue en plan de la portion d'une poche proche de son ouverture, selon une variante de réalisation, particulièrement adaptée à l'usage comme urinal féminin ;
- la figure 6 est une vue en perspective du manchon de la poche de la figure 5 ; et
- la figure 7 est une vue en perspective montrant la forme du manchon en configuration d'utilisation de la poche de la figure 5.

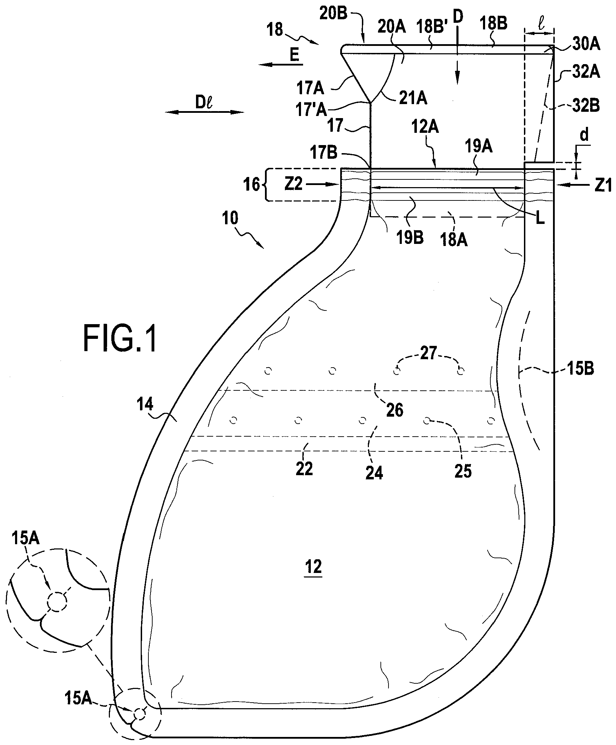

La poche 10 représentée sur la figure 1 comprend une enveloppe souple 12. En particulier, cette enveloppe est réalisée à partir de deux feuilles minces de matière plastique découpées de manière convenable et soudées l'une à l'autre sur la quasi-totalité du contour, par une ligne de soudure 14. L'enveloppe 12 présente une ouverture 12A ménagée par une interruption de la ligne de soudure 14, pour permettre à l'espace intérieur de l'enveloppe de communiquer avec l'extérieur.

Dans l'exemple représenté, cette ouverture est ménagée à l'extrémité libre d'une portion de col 16 que présente l'enveloppe, dans laquelle la largeur de cette enveloppe est réduite.

La poche comprend un manchon de renfort 18 qui est fixé en travers de l'ouverture 12A et fait saillie à l'extérieur de l'enveloppe.

En l'espèce, le manchon comprend non seulement sa partie principale de manchon proprement dit, qui fait saillie au-delà de l'ouverture 12, mais également une portion interne 18A, qui est insérée dans l'ouverture de manière à s'étendre sur toute la largeur L de cette dernière.

Par exemple, les feuilles qui forment l'enveloppe sont en matière plastique souple, tandis que le manchon est en matière plastique plus rigide. Ainsi, pour opérer la fixation du manchon avec l'enveloppe, il suffit de souder ou coller les feuilles de l'enveloppe aux parois du manchon. On voit ainsi sur la figure 1 deux lignes de soudure, respectivement 19A et 19B orientées transversalement à la direction D d'introduction des déchets dans la poche, lignes sur lesquelles la paroi avant 20A du manchon, visible sur la figure 1, est soudée à la feuille correspondante de l'enveloppe. Bien entendu, la paroi arrière 20B du manchon est soudée de la même manière à la feuille opposée de la poche.

On constate également sur la figure 1 que, dans une région opposée à l'ouverture, la ligne de soudure 14 présente une ligne d'affaiblissement 15A, réalisée par exemple à l'aide de micro perçages en pointillées. Cet affaiblissement s'étend seulement sur la ligne de soudure, sans atteindre l'espace interne de l'enveloppe. Lorsque la poche est pleine de déchets, ceux-ci peuvent ainsi être vidés en déchirant l'enveloppe selon la ligne d'affaiblissement 15A, avec un effort suffisant pour que la déchirure ainsi créée s'étende jusqu'à l'espace interne de l'enveloppe.

Par ailleurs, sur l'un de ces côtés voisins de l'ouverture, la ligne de soudure 14 présente une autre ligne d'affaiblissement 15B, pouvant être également réalisée par des perçages discontinus. Cette ligne est à distance de l'espace interne de l'enveloppe et elle est orientée de telle sorte que sa déchirure ne peut pas se propager jusqu'à l'intérieur de l'enveloppe. Ainsi, lorsque la poche est pleine et que l'on souhaite la stocker avant de la vider, la ligne 15B peut être déchirée pour ménager une fente permettant d'accrocher la poche à un crochet ou analogue.

On voit encore sur la figure 1 que la poche présente un clapet de sécurité du type décrit dans la demande de brevet européen n° 0 847 742. Ainsi, ce clapet est constitué de plusieurs feuilles internes disposées contre chacune des parois de la poche. Dans l'exemple représenté, le clapet comporte, pour chacune des deux parois de la poche, une première paire de feuilles 22 qui s'étend à partir de l'ouverture 12A jusqu'à une région médiane de la poche, une deuxième paire de feuilles 24 qui s'étend à l'intérieur des feuilles de la première paire 22, et une troisième paire de feuilles 26 qui s'étend à l'intérieur de la paire de feuilles 24, les feuilles 26 étant plus courtes que les feuilles 24, elles-mêmes plus courtes que les feuilles 22. Ces feuilles s'étendent sur toute la largeur de la poche dans la région où elles se trouvent, et sont donc soudées entre elles et avec les parois de l'enveloppe, par la ligne de soudure 14. Par ailleurs, les feuilles de la paire 24 sont soudées entre elles par des points de soudure 25, et les feuilles des paires 26 et 24 sont soudées par des points de soudure 27 décalés par rapport aux points de soudure 25. Ces différentes paires de feuilles astucieusement soudées constituent un clapet anti-reflux, qui empêche le reflux de déchets cités dans la poche lorsque celle-ci est manipulée après son utilisation.

On constate que la ligne d'affaiblissement 15B se trouve dans une région de la ligne de soudure dans laquelle tout ou partie des différentes couches de feuilles sont présentes, de sorte que cette soudure est particulièrement résistante, ceci convenant à l'accrochage précédemment évoqué.

L'invention s'intéresse tout particulièrement au manchon 18. Comme indiqué précédemment, il est soudé en travers de l'ouverture, sa partie interne 18A s'étendant sur toute la largeur L de l'ouverture 12A. Sur la figure 1, qui est une vue de côté, le manchon 18 est aplati et on voit sa paroi avant 20A, ci-après dénommée "première paroi", tandis que sa deuxième paroi 20B est cachée par la première paroi.

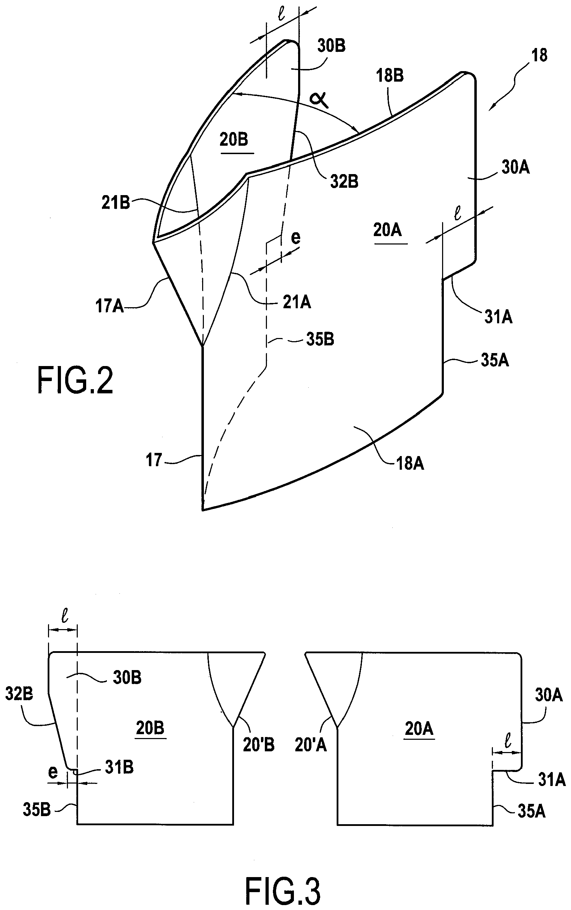

En se reportant également à la figure 2, on comprend que les deux parois 20A et 20B sont reliées par une ligne de pliage principale 17. On voit en outre que cette ligne de pliage principale 17 présente une portion d'extrémité 17A (son extrémité opposée à l'ouverture 12A) qui est en avancée latérale vers l'extérieur. Ici, le sens « vers l'extérieur » est le sens indiqué par la flèche E de la figure 1, qui va en s'éloignant des bords longitudinaux du manchon opposés à la ligne de pliage principale 17, étant précisé que, au sens de la présente demande de brevet, la direction longitudinale est la direction D d'introduction des déchets dans la poche, elle-même définie par l'axe du manchon 18.

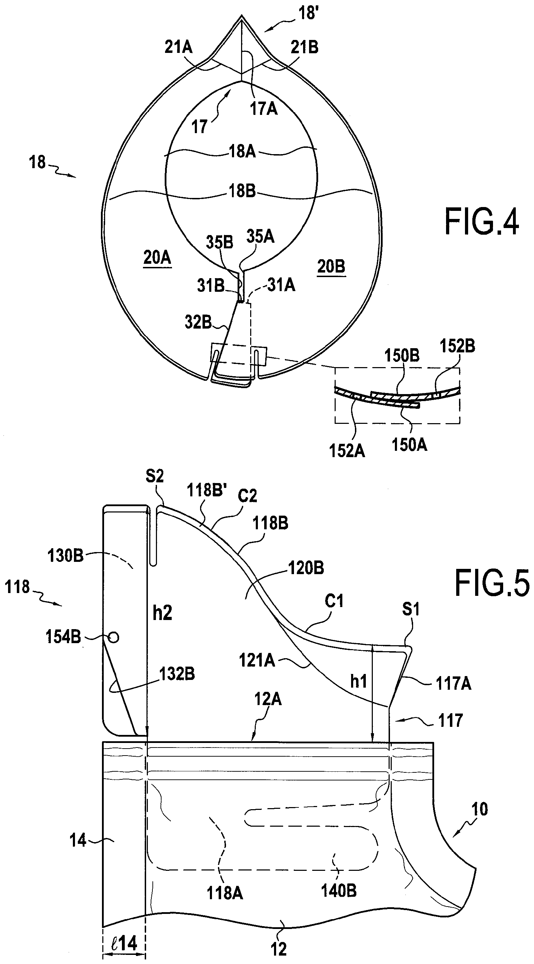

On note que la portion d'extrémité 17A est en avancée latérale vers l'extérieur lorsque la poche est dans sa configuration aplatie (comme illustré sur la figure 1) et lorsque la poche est dans sa configuration d'utilisation (comme illustré sur la figure 4).

Sur la figure 4, qui montre la forme du manchon lorsque la poche est dans sa configuration d'utilisation, on voit que, dans cette configuration d'utilisation, cette portion en avancée 17A est conformée en bec 18'.

Comme on le voit en particulier sur les figures 1 et 2, la ligne de pliage principale 17 présente une concavité tournée vers l'extérieur du manchon, c'est-à-dire dans le sens de la flèche E. En l'espèce, la base 17B de la ligne de pliage principale 17 est sensiblement rectiligne, de direction longitudinale, et la ligne de pliage s'infléchit pour présenter la portion en avancée 17A, également sensiblement rectiligne. Dans l'exemple représenté, l'inflexion 17'A forme un angle et les deux segments de la ligne de pliage qui sont situés de part et d'autre de cette inflexion sont rectiligne. On pourrait toutefois prévoir une inflexion plus douce et/ou des segments légèrement courbes.

Dans l'exemple représenté, chacune des parois 20A et 20B du manchon présente une ligne de pliage secondaire, respectivement 21A et 21B. Sur chaque paroi, la ligne de pliage secondaire, 21A ou 21B, relie la ligne de pliage principale 17 au bord supérieur 18B du manchon 18, c'est-à-dire au bord libre du manchon qui est opposé à l'ouverture 12A de la poche.

Ces lignes de pliage secondaires favorisent la mise en volume du bec 18', lorsque le manchon passe de sa configuration aplatie à sa configuration d'utilisation. En effet, les lignes de pliage secondaires 21A et 21B forment des démarcations entre le bec 18' et le reste du manchon. Lorsque la poche passe dans sa configuration d'utilisation, le manchon est conformé en canal et ses parois 18A et 18B ont naturellement tendance à s'incurver selon les lignes de pliage secondaires 21A et 21B, et le bec adopte naturellement sa forme.

On voit notamment sur les figures 2 et 4 que les lignes de pliage secondaires 21A et 21B sont symétriques par rapport à la ligne de pliage principale 17. Par ailleurs, dans l'exemple représenté, les lignes de pliage secondaires sont courbes, de concavité tournée vers la ligne de pliage principale. Elles pourraient toutefois être rectilignes, en général en biais par rapport à la direction longitudinale, ou avoir des tronçons rectilignes.

On comprend que le bec 18' favorise le guidage des produits, en particulier de l'urine, dans le manchon et, par conséquent, dans la poche. Du fait de la conformation de la ligne de pliage principale 17, et de celle des lignes de pliage secondaires lorsqu'elles sont présentes, le bec se forme naturellement lorsque le manchon est conformé en canal pour utiliser la poche. On relève que la présence du bec n'empêche nullement la poche et le manchon d'adopter une configuration parfaitement aplatie pour le stockage.

On décrit maintenant un autre aspect du manchon 18. On remarque en effet que la paroi avant 20A du manchon présente une première bande longitudinale d'extrémité 30A qui est en saillie latérale par rapport au bord de l'ouverture 12A. Cette première bande longitudinale 30A dépasse latéralement d'une largeur I par rapport au bord de l'ouverture, selon la direction latérale DI perpendiculaire à la direction longitudinale D. En se reportant à la figure 2, on comprend que la partie interne 18A du manchon est délimitée par rapport à la partie de ce manchon qui dépasse en dehors de l'ouverture lorsque le manchon est en place dans la poche, par un décrochement 31A de largeur l.

De son côté, la deuxième paroi 20B du manchon présente un bord de glissement 32B qui est apte à glisser contre la face interne de la première bande 30A lors du passage de la poche de sa configuration aplatie à sa configuration d'utilisation.

Pour provoquer le changement de forme du manchon et parvenir à la situation de la figure 4, l'utilisateur exerce une pression dans les zones Z1 et Z2 indiquées sur la figure 1, situées à la base du manchon.

Sous l'effet de cette pression qui tend à rapprocher l'un de l'autre les bords longitudinaux opposés du manchon, les parois du manchon se courbent, de manière à le conformer en canal.

Dans le même temps, la zone Z1 étant située sous le bec, celui-ci se forme en prenant la forme d'un bec verseur.

Plus précisément, les parois 20A, 20B du manchon se plient le long des lignes de pliage secondaires 21A, 21B comme cela a été indiqué précédemment. Les parties des parois du manchon situées entre les lignes de pliage secondaires 21A, 21B et la ligne de pliage principale 17 (en haut de la Figure 4) s'écartent l'une de l'autre tout en restant en avancée latérale vers l'extérieur, ce qui donne au bec 18' la forme d'un bec verseur.

La bande longitudinale 30A et le bord de glissement 32B facilitent la mise en forme du manchon en canal. En effet, sous l'effet de la pression dans les zones Z1 et Z2, la première paroi 20A adopte une forme convexe tandis que sa bande longitudinale d'extrémité, qui forme une extension libre de cette première paroi, adopte également une forme convexe mais avec une courbure moindre. La face intérieure de la première paroi 20A exerce un effort de réaction contre le bord libre 32B et, du fait de la forme particulière adoptée par la première bande longitudinale 30A, le bord de glissement n'a d'autre choix que de glisser sur cette face interne, pour conformer la deuxième paroi 20B selon une forme convexe. Ainsi, les deux parois délimitent entre elles le canal provoquant le maintien de l'ouverture de la poche en situation ouverte.

En l'espèce, le bord de glissement 32B est formé sur le bord d'une deuxième bande longitudinale d'extrémité 30B qui fait partie de la deuxième paroi du manchon et est en saillie latérale par rapport au bord de l'ouverture. Sur au moins un tronçon, la bande 30B dépasse moins latéralement que la bande 30A. En l'espèce, le dépassement latéral de la bande 30B varie du fait de l'inclinaison du bord de glissement 32B entre la valeur minimale e, sur le décrochement 31B situé à la base du bord de glissement 32B, et la valeur maximale I, sur le bord libre du manchon.

La figure 2 montre le manchon formant une pièce, ses parois 20A et 20B étant reliées par la ligne de pliage 17. Ce manchon peut être obtenu en une seule pièce, par exemple par moulage par injection. Dans ce cas, l'angle d'ouverture α entre ses deux parois peut être de l'ordre de 30 à 90°, de préférence de l'ordre de 60°, pour faciliter le démoulage. Les lignes de pliage 17, 21A et 21B sont avantageusement formées par des diminutions locales d'épaisseur de la matière du manchon.

Comme l'indique la figure 3, on peut également prévoir de réaliser le manchon à partir de deux pièces plates, c'est-à-dire sous la forme de deux flancs distincts formant respectivement les parois 20A et 20B, soudés entre eux selon leurs bords respectifs 20'A et 20'B, de sorte que ces bords soudés forment la ligne de pliage. la soudure est alors réalisée de manière à favoriser le pliage, en particulier en évitant de former des surépaisseurs locales.

Les bords du manchon opposés à la ligne de pliage principale 17, sont libres de se déplacer l'un par rapport à l'autre. Ceci concerne toute la partie du manchon qui fait saillie au-delà de l'ouverture 12A. En effet, pour ce qui concerne la partie interne 18A du manchon, on a indiqué précédemment que la largeur L de cette partie interne en configuration aplatie correspond à celle de l'ouverture 12A. Ainsi, pour cette partie interne, les bords 35A et 35B sont en fait incapables ou pratiquement incapables de se déplacer l'un par rapport à l'autre, du fait de la présence de la ligne de soudure 14. Sur les côtés de l'ouverture 12A, cette ligne 14 peut d'ailleurs légèrement empiéter sur le manchon c'est-à-dire sur la partie de la ligne de pliage principale 17 qui s'étend sur la partie interne 18A du manchon, et sur les bords 35A et 35B précédemment évoqués.

Dans l'exemple représenté sur les figures 1 à 4, la hauteur de dépassement du manchon par rapport à l'ouverture est sensiblement constante. Bien entendu, ceci n'empêche pas que le bord supérieur 18B du manchon 18 puisse légèrement être adouci si nécessaire. Ainsi, il peut être chanfreiné ou bien pourvu d'un bourrelet 18B'.

En référence aux figures 5 à 7, on décrit maintenant une variante de réalisation du manchon. Sur la figure 5, on a seulement représenté la partie de la poche proche de son ouverture 12A. Le manchon 118 est analogue au manchon 18 précédemment décrit mais il s'en distingue en deux aspects. D'une part, le bord libre 118B du manchon 118 opposé à l'ouverture 12A a une conformation particulière, en particulier adaptée à son utilisation comme urinale pour les femmes. On relève d'ores et déjà que ce bord libre peut présenter un bourrelet ou analogue 118B' le rendant agréable au toucher. D'autre part, la partie interne 118A du manchon présente des pattes d'écartement qui seront décrites ci-après.

Les différents éléments constitutifs du manchon 118 sont désignés par les mêmes références que pour le manchon 18, augmentées de 100. En se référant aux figures 5 à 7, on voit donc que le manchon 118 présente une première paroi 120A et une deuxième paroi 120B, reliées par une ligne de pliage principale 117 ayant une portion d'extrémité 117A en avancée. Les parois présentent chacune une ligne de pliage secondaire, respectivement 121A et 121B.

De plus, la première paroi 120A présente une première bande longitudinale d'extrémité 130A, tandis que la deuxième paroi 120B présente un bord de glissement 132B ménagé sur le bord d'une deuxième bande longitudinale 130B. Les deux bandes longitudinales dépassent latéralement de l'ouverture 12A. La première bande longitudinale 130A se situe globalement dans le prolongement de la ligne de soudure 14 et présente une largeur correspondant approximativement à celle de cette ligne de soudure. La deuxième bande longitudinale 130B présente une portion terminale libre 130B' de largeur sensiblement constante, correspondant à la largeur 114 de la ligne de soudure 14. Entre le bord de l'ouverture 12A et cette portion terminale 130B', cette deuxième bande longitudinale d'extrémité présente le bord de glissement 132B qui, comme le bord de glissement 32B de la précédente variante, est incliné pour mieux glisser contre la face interne de la première bande longitudinale 130A. Des décrochements 131A et 131B, respectivement analogues aux décrochements 31A et 31B précédemment évoqués, sont situés à la base des bandes longitudinales 130A et 130B.

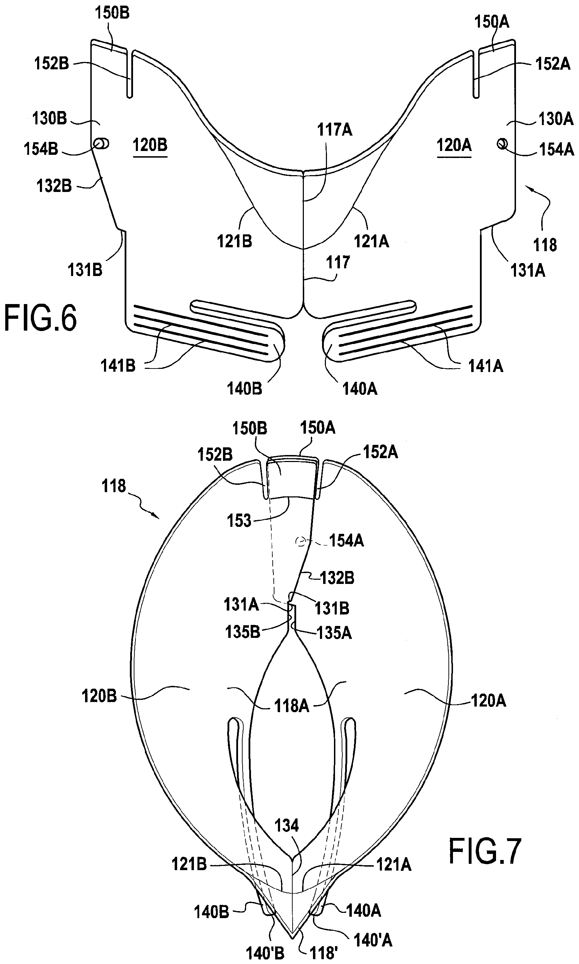

Comme le manchon 18, le manchon 118 peut être réalisé à partir de deux flancs plats soudés entre eux, ou bien, sous la forme d'une seule pièce obtenue de moulage, telle que montrée par la figure 6.

La hauteur de dépassement du manchon 118 par rapport à l'ouverture 12A varie sur la largeur du manchon, entre une hauteur minimale h1 mesurée au voisinage de la ligne de pliage principale 117, et une hauteur maximale h2 mesurée au voisinage de l'extrémité latérale du manchon opposée à la ligne de pliage principale, c'est-à-dire, en l'espèce, l'extrémité à laquelle se trouvent la première bande longitudinale d'extrémité 130A et le bord de glissement 132B.

Comme on le voit sur la figure 5, en configuration aplatie de la poche, le bord libre 118B du manchon opposé à l'ouverture 12A forme une courbe avec une zone concave C1 qui délimite au moins une partie du bord libre du bec 118'. Cette courbe présente en outre une zone convexe C2 qui s'étend entre la zone concave C1 et l'extrémité latérale du manchon opposée à la ligne de pliage principale 117.

En fait, cette courbe présente sensiblement une forme en S avec une portion centrale en forme de rampe montante qui relie les portions C1 et C2. Ses portions terminales respectivement S1 et S2 sont légèrement arrondies. Cette forme est particulièrement adaptée à l'usage de la poche comme urinal pour les femmes. Pour utiliser la poche, il suffit de déformer le manchon pour le conformer en canal, et de placer ce manchon de telle sorte que la partie de plus petite hauteur, correspondant à la portion S2, se trouve contre la partie postérieure de la vulve.

Dans la variante des figures 5 à 7, le manchon présente en outre des pattes d'écartement. Plus précisément, la partie 118A du manchon qui forme un tronçon de ce dernier interne à l'enveloppe 12, présente des pattes d'écartement 140A et 140B. Ces pattes sont respectivement formées dans la continuité de chacune des deux parois du manchon et ont des extrémités latérales libres, respectivement 140'A et 140'B qui sont aptes à s'écarter l'une de l'autre dans la configuration d'utilisation de la poche pour espacer les faces opposées de l'enveloppe souple, comme décrit dans la demande de brevet européen n° 0 847 742 et comme on le comprend en considérant la figure 6. Ces pattes sont avantageusement rigidifiées par des nervures 141A et 141B ou analogues.

La poche selon l'invention comprend avantageusement des moyens pour maintenir les parois du manchon dans leur conformation formant un canal.

Ainsi, le manchon 118 présente deux languettes de maintien, respectivement 150A et 150B, respectivement portées par la première paroi 120A et par la deuxième paroi 120B. Ces languettes sont situées vers les extrémités latérales des parois qui sont éloignées de la ligne de pliage principale 117 et elles s'étendent vers le bord libre du manchon, c'est-à-dire du côté opposé à l'ouverture 12A de la poche.

Plus précisément, pour chaque paroi 120A, 120B, la languette de maintien 150A, 150B s'étend entre le bord de la paroi opposé à la ligne de pliage principale 117 (bord qui, en l'espèce, porte la bande 130A ou le bord de glissement 132B) et une entaille, respectivement 152A, 152B. L'entaille est orientée longitudinalement vers l'ouverture 12A de la poche à partir du bord libre 118B du manchon.

Comme on le voit sur la figure 7, les languettes 150A et 150B se recouvrent lorsque la poche est en configuration d'utilisation, alors que le manchon forme le canal. Pour maintenir le manchon en canal, on peut alors saisir les deux languettes se recouvrant, en les pinçant entre deux doigts. D'ailleurs, pour favoriser la stabilité de la conformation du manchon en canal, on peut replier les languettes vers l'extérieur, selon la ligne de flexion 153 visible sur la figure 7.

Bien entendu, les languettes de maintien sont parfaitement compatibles avec la variante de réalisation des figures 1 à 4.

Les figures 5 à 7 montrent également un autre moyen de maintien des parois du manchon dans leur configuration formant un canal. En effet, la deuxième portion de bande longitudinale 130B porte un ergot 154B en saillie vers l'extérieur, tandis que la première portion de bande longitudinale 130A présente un perçage 154A destiné à recevoir cet ergot lorsque le manchon est conformé en canal, pour maintenir le manchon dans cette conformation.

De manière générale, l'ergot et le perçage sont respectivement situés dans deux parties du manchon qui viennent en recouvrement lorsque les parois du manchon sont dans leur conformation en canal, ces deux parties étant de préférence éloignées de la ligne de pliage principale. Ainsi, lorsqu'elles sont présentes, ces deux parties peuvent être les deux bandes longitudinales, comme représenté.

On peut prévoir que l'ergot coopère avec l'ouverture par clippage ou encliquetage, ou part tout sytème de retenue approprié. Par exemple, l'ergot pourrait avoir une tête de retenue et l'ouverture pourrait avoir une forme en trou de serrure, avec une partie large d'introduction et une partiie étroite de retenue.

Les moyens de maintien qui comprennent les languettes précédemment décrites, ainsi que ceux qui comprennent l'ergot et l'ouverture, peuvent être combinés, comme sur les figures 5 à 7, ou bien seul l'un ou l'autre de ces moyens peut être prévu.

De manière générale, tous moyens de maintien du manchon dans sa conformation en canal peuvent être prévus, qu'il s'agisse des moyens qui viennent d'être décrit ou d'autres moyens, par exemple faisant appel à un collage ou analogue.

## Revendications

1. Poche (10) comprenant une enveloppe souple (12) ayant une ouverture (12A) dont le bord est fixé aux deux parois opposées d'un manchon de renfort (18 ; 118) en saillie à l'extérieur de l'enveloppe, la poche étant susceptible d'adopter une configuration aplatie dans laquelle le manchon est plié selon une ligne de pliage principale (17 ; 117) reliant ses parois de sorte que lesdites parois sont situées l'une contre l'autre, et une configuration d'utilisation dans laquelle ces parois forment un canal qui maintient l'ouverture ouverte,
dans laquelle la ligne de pliage principale (17 ; 117) présente une portion d'extrémité (17A ; 117A) opposée à l'ouverture qui est en avancée latérale vers l'extérieur, de sorte que, en configuration d'utilisation, le manchon présente un bec (18'; 118') délimité par ladite portion d'extrémité, et **caractérisée en ce que** la ligne de pliage principale (17 ; 117) présente une concavité tournée vers l'extérieur du manchon (18 ; 118).

2. Poche selon la revendication 1, **caractérisée en ce que** chacune des parois (20A, 20B ; 120A, 120B) du manchon (18 ; 118) présente une ligne de pliage secondaire (21A, 21B ; 121A, 121B), qui relie la ligne de pliage principale (17 ; 117) au bord libre (18B ; 118B) du manchon opposé à l'ouverture (12A) et qui, en configuration d'utilisation, forme une démarcation entre le bec (18' ; 118') et une partie restante du manchon.

3. Poche selon la revendication 2, **caractérisée en ce que** les lignes de pliage secondaires (21A, 21B ; 121A, 121B) sont symétriques par rapport à la ligne de pliage principale (17 ; 117).

4. Poche selon la revendication 2 ou 3, **caractérisée en ce que** les lignes de pliage secondaires (21A, 21B ; 121A, 121B) sont courbes, de concavité tournée du côté de la ligne de pliage principale (17 ; 117).

5. Poche selon l'une quelconque des revendications 1 à 4, **caractérisée en ce que** la hauteur de dépassement du manchon (118) par rapport à l'ouverture (12A) varie sur la largeur du manchon, entre une hauteur minimale (h1) mesurée au voisinage de la ligne de pliage principale (117) et une hauteur maximale (h2) mesurée au voisinage de l'extrémité latérale du manchon opposée à la ligne de pliage principale.

6. Poche selon la revendication 5, **caractérisée en ce que**, en configuration aplatie de la poche, le bord libre (118B) du manchon (118) opposé à l'ouverture (12A), forme une courbe ayant une zone concave (C1) qui délimite au moins une partie du bord libre du bec (118').

7. Poche selon la revendication 6, **caractérisé en ce que** la courbe a une zone convexe (C2) qui s'étend entre ladite zone concave et l'extrémité latérale du manchon (118) opposée à la ligne de pliage principale (117).

8. Poche selon l'une quelconque des revendications 1 à 7, **caractérisée en ce que**, du côté opposé à la ligne de pliage principale, la première paroi (20A ; 120A) du manchon (18 ; 118) présente une première bande longitudinale d'extrémité (30A ; 130A) en saillie latérale par rapport au bord de l'ouverture, tandis que la deuxième paroi (20B ; 120B) présente un bord de glissement (32B ; 132B) apte à glisser contre la face interne de ladite première bande longitudinale lors du passage de la poche de sa configuration aplatie à sa configuration d'utilisation.

9. Poche selon l'une quelconque des revendications 1 à 8, **caractérisée en ce qu'**elle comprend des moyens (150A, 150B) pour maintenir les parois du manchon (118) dans leur conformation formant un canal.

10. Poche selon l'une quelconque des revendications 1 à 9, **caractérisée en ce que** le manchon (118) comporte deux languettes de maintien (150A, 150B), respectivement portées par la première et par la deuxième paroi (120A, 120B) du manchon, du côté opposé à l'ouverture (12A) de la poche et dans une zone éloignée de la ligne de pliage principale (117), les languettes de maintien se recouvrant au moins partiellement dans la position d'utilisation.

11. Poche selon la revendication 10, **caractérisée en ce que**, pour chaque paroi (120A, 120B), la languette de maintien (150A, 150B) s'étend entre un bord de la paroi opposé à la ligne de pliage principale (117) et une entaille (152A, 152B) qui s'étend sensiblement longitudinalement vers l'ouverture (12A) à partir du bord libre (118B) du manchon (118).

12. Poche selon l'une quelconque des revendications 1 à 11, **caractérisée en ce que** deux parties (130A, 130B) du manchon, destinées à venir en recouvrement lorsque les parois (120A, 120B) du manchon sont dans leur conformation formant un canal, présentent respectivement un perçage (154A) et un ergot (154B) apte à être engagé dans ledit perçage.

13. Poche selon la revendication 12, **caractérisée en ce que** les deux parties (130A, 130B) destinées à venir en recouvrement sont avantageusement situées dans des zones respectives de la première et de la deuxième paroi (120A, 120B) du manchon qui sont éloignées de la ligne de pliage principale (117).

14. Poche selon l'une quelconque des revendications 1 à 13, **caractérisée en ce que** la portion d'extrémité (17A ; 117A) est en avancée latérale vers l'extérieur lorsque la poche (10) est dans sa configuration aplatie et lorsque la poche (10) est dans sa configuration d'utilisation.

## Patentansprüche

1. Beutel (10) umfassend eine flexible Hülle (12) mit einer Öffnung (12A), deren Rand an den beiden gegenüberliegenden Wänden einer Verstärkungsmanschette (18; 118), welche außerhalb der Hülle vorspringt, befestigt ist, wobei der Beutel in der Lage ist, eine flach gelegte Ausführung, in der die Manschette entlang einer Hauptfaltlinie (17; 117), welche ihre Wände verbindet, gefaltet ist, so dass die Wände aneinander liegen, sowie eine Gebrauchsausführung anzunehmen, in der diese Wände einen Kanal bilden, der die Öffnung offen hält,
wobei die Hauptfaltlinie (17; 117) einen der Öffnung gegenüberliegenden Endabschnitt (17A; 117A), der seitlich nach außen vorspringt, aufweist, so dass die Manschette in der Gebrauchsausführung einen durch den Endabschnitt begrenzten Ausguss (18'; 118') aufweist, und **dadurch gekennzeichnet, dass** die Hauptfaltlinie (17; 117) eine zur Außenseite der Manschette (18; 118) gewandte Konkavität aufweist.

2. Beutel nach Anspruch 1, **dadurch gekennzeichnet, dass** eine jede der Wände (20A, 20B; 120A, 120B) der Manschette (18; 118) eine sekundäre Faltlinie (21 A, 21 B; 121A, 121 B) aufweist, welche die Hauptfaltlinie (17; 117) mit dem freien Rand (18B; 118B) der Manschette, welcher der Öffnung (12A) gegenüberliegt, verbindet und welche in der Gebrauchsausführung eine Abgrenzung zwischen dem Ausguss (18'; 118') und einem restlichen Teil der Manschette bildet.

3. Beutel nach Anspruch 2, **dadurch gekennzeichnet, dass** die sekundären Faltlinien (21 A, 21 B; 121A, 121 B) in Bezug auf die Hauptfaltlinie (17; 117) symmetrisch sind.

4. Beutel nach Anspruch 2 oder 3, **dadurch gekennzeichnet, dass** die sekundären Faltlinien (21 A, 21 B; 121A, 121 B) mit einer von Seiten der Hauptfaltline (17; 117) gewandten Konkavität gekrümmt sind.

5. Beutel nach einem der Ansprüche 1 bis 4, **dadurch gekennzeichnet, dass** die Überschreitungshöhe der Manschette (118) gegenüber der Öffnung (12A) über die Breite der Manschette zwischen einer in der Nähe der Hauptfaltlinie (117) gemessenen minimalen Höhe (h1) und einer in der Nähe des der Hauptfaltlinie gegenüberliegenden seitlichen Endes der Manschette gemessenen maximalen Höhe (h2) variiert.

6. Beutel nach Anspruch 5, **dadurch gekennzeichnet, dass** in der flach gelegten Ausführung des Beutels der der Öffnung (12A) gegenüberliegende freie Rand (118B) der Manschette (118) eine Kurve mit einem konkaven Bereich (C1), welcher wenigstens einen Teil des freien Randes des Ausgusses (118') begrenzt, bildet.

7. Beutel nach Anspruch 6, **dadurch gekennzeichnet, dass** die Kurve einen konvexen Bereich (C2) aufweist, der sich zwischen dem konkaven Bereich und dem der Hauptfaltlinie (117) gegenüberliegenden seitlichen Ende der Manschette (118) erstreckt.

8. Beutel nach einem der Ansprüche 1 bis 7, **dadurch gekennzeichnet, dass** auf der der Hauptfaltlinie gegenüberliegenden Seite die erste Wand (20A; 120A) der Manschette (18; 118) einen ersten endseitigen Längsstreifen (30A; 130A), welcher gegenüber dem Rand der Öffnung seitlich vorspringt, aufweist, während die zweite Wand (20B; 120B) einen Gleitrand (32B; 132B) aufweist, der geeignet ist, beim Übergang des Beutels von seiner flach gelegten Ausführung in seine Gebrauchsausführung an der Innenseite des ersten Längsstreifens zu gleiten.

9. Beutel nach einem der Ansprüche 1 bis 8, **dadurch gekennzeichnet, dass** er Mittel (150A, 150B) umfasst, um die Wände der Manschette (118) in ihrer einen Kanal bildenden Gestalt zu halten.

10. Beutel nach einem der Ansprüche 1 bis 9, **dadurch gekennzeichnet, dass** die Manschette (118) zwei Haltelaschen (150A, 150B), die durch die erste bzw. durch die zweite Wand (120A, 120B) der Manschette getragen sind, auf der der Öffnung (12A) des Beutels gegenüberliegenden Seite und in einem von der Hauptfaltlinie (117) entfernten Bereich umfasst, wobei die Haltelaschen sich in der Gebrauchsstellung wenigstens teilweise überlappen.

11. Beutel nach Anspruch 10, **dadurch gekennzeichnet, dass** bei jeder Wand (120A, 120B) die Haltelasche (150A, 150B) zwischen einem von der Hauptfaltlinie (117) abgewandten Rand der Wand und einem Einschnitt (152A, 152B), welcher sich von dem freien Rand (118B) der Manschette (118) aus im Wesentlichen längs zur Öffnung (12A) erstreckt, verläuft.

12. Beutel nach einem der Ansprüche 1 bis 11, **dadurch gekennzeichnet, dass** zwei Teile (130A, 130B) der Manschette, welche dazu bestimmt sind, sich zu überlappen, wenn die Wände (120A, 120B) der Manschette sich in ihrer einen Kanal bildenden Gestalt befinden, eine Bohrung (154A) bzw. einen Stift (154B), der geeignet ist, in die Bohrung eingesteckt zu werden, aufweisen.

13. Beutel nach Anspruch 12, **dadurch gekennzeichnet, dass** die beiden Teile (130A, 130B), welche dazu bestimmt sind, sich zu überlappen, vorteilhafterweise in jeweiligen Bereichen der ersten und der zweiten Wand (120A, 120B) der Manschette, die von der Hauptfaltlinie (117) entfernt sind, gelegen sind.

14. Beutel nach einem der Ansprüche 1 bis 13, **dadurch gekennzeichnet, dass** der Endabschnitt (17A; 117A) seitlich nach außen vorspringt, wenn der Beutel (10) sich in seiner flach gelegten Ausführung befindet und wenn der Beutel (10) sich in seiner Gebrauchsausführung befindet.

## Claims

1. A pouch (10) comprising a flexible bag (12) having an opening (12A) whose edge is fastened to two opposite walls of a reinforcing collar (18; 118) projecting out from the bag, the pouch being able to adopt a flat configuration in which the collar is folded along a main fold line (17; 117) connecting together its walls so that said walls are situated one against the other, and a utilization configuration in which the walls form a channel holding the opening open,
wherein the main fold line (17; 117) presents an end portion (17A; 117A) opposite from the opening that projects laterally outwards, such that in the utilization configuration, the collar presents a nose (18'; 118') defined by said end portion, and **characterized in that** the main fold line (17; 117) presents a concave side facing towards the outside of the collar (18; 118).

2. A pouch according to claim 1, **characterized in that** each of the walls (20A, 20B; 120A, 120B) of the collar (18; 118) presents a secondary fold line (21A, 21B; 121A, 121B) that connects the main fold line (17; 117) to the free edge (18B; 118B) of the collar opposite from the opening (12A), and that, in the utilization configuration, forms a boundary between the nose (18'; 118') and a remaining portion of the collar.

3. A pouch according to claim 2, **characterized in that** the secondary fold lines (21A, 21B; 121A, 121B) are symmetrical relative to the main fold line (17; 117).

4. A pouch according to claim 2 or claim 3, **characterized in that** the secondary fold lines (21A, 21B; 121A, 121B) are curved, with concave sides facing towards the main fold line (17; 117).

5. A pouch according to any one of claims 1 to 4, **characterized in that** the extent to which the collar (118) projects from the opening (12A) varies across the width of the collar, between a minimum height (h1) measured in the vicinity of the main fold line (117) and a maximum height (h2) measured in the vicinity of the lateral end of the collar opposite from the main fold line.

6. A pouch according to claim 5, **characterized in that**, in the flat configuration of the pouch, the free edge (118B) of the collar (118) opposite from the opening (12A) forms a curve having a concave zone (C1) that defines at least a portion of the free edge of the nose (118').

7. A pouch according to claim 6, **characterized in that** the curve has a convex zone (C2) that extends between said concave zone and the lateral end of the collar (118) that is opposite from the main fold line (117).

8. A pouch according to any one of claims 1 to 7, **characterized in that**, at its side opposite from the main fold line, the first wall (20; 120A) of the collar (18; 118) presents a first longitudinal end strip (30A; 130A) projecting laterally relative to the edge of the opening, while the second wall (20B; 120B) presents a sliding edge (32B; 132B) suitable for sliding against the inside face of said first longitudinal strip while the pouch is passing from its flat configuration to its utilization configuration.

9. A pouch according to any one of claims 1 to 8, **characterized in that** it includes means (150A, 150B) for holding the walls of the collar (118) in their channel-forming shape.

10. A pouch according to any one of claims 1 to 9, **characterized in that** the collar (118) presents two holder tongues (150A, 150B) carried respectively by the first and second walls (120A, 120B) of the collar, on the side opposite from the opening (12A) of the pouch and in a zone that is remote from the main fold line (117), the holder tongues overlapping at least in part in the utilization position.

11. A pouch according to claim 10, **characterized in that**, for each wall (120A, 120B), the holder tongue (150A, 150B) extends between an edge of the wall opposite from the main fold line (117) and a notch (152A, 152B) that extends substantially longitudinally towards the opening (12A) from the free edge (118B) of the collar (118).

12. A pouch according to any one of claims 1 to 11, **characterized in that** two portions (130A, 130B) of the collar, that are to move into overlap when the walls (120A, 120B) of the collar are in their channel-forming shape, present respectively a hole (154A) and a lug (154B) suitable for being engaged in said hole.

13. A pouch according to claim 12, **characterized in that** the two portions (130A, 130B) that are to move into overlap are advantageously situated in respective zones of the first and second walls (120A, 120B) of the collar that are remote from the main fold line (117).

14. A pouch according to any one of claims 1 to 13, **characterized in that** the end portion (17A; 117A) projects laterally outwards when the pouch (10) is in its flat configuration and when the pouch (10) is in its utilization configuration.
